# FASCICULE DE BREVET EUROPEEN

(11) **EP 3 060 413 B1**
(45) Date de publication et mention de la délivrance du brevet: **05.08.2020**
(21) Numéro de dépôt: 14789821.7
(22) Date de dépôt: 24.10.2014
(51) Int. Cl.: B60C 1/00, C07D 233/64, C08C 19/22, C08K 5/32, C08K 5/3445

(54) **COMPOSITION DE CAOUTCHOUC COMPRENANT UN ADDITIF COMPOSE 1,3-DIPOLAIRE PORTANT UNE FONCTION IMIDAZOLE**
KAUTSCHUKZUSAMMENSETZUNG MIT EINEM ADDITIV AUS EINER 1,3-DIPOLAREN VERBINDUNG MIT IMIDAZOLFUNKTION
RUBBER COMPOSITION COMPRISING A 1,3-DIPOLAR COMPOUND ADDITIVE HAVING AN IMIDAZOLE FUNCTION

(30) Priorité: 25.10.2013 FR 1360415
(43) Date de publication de la demande: 31.08.2016
(73) Titulaire: Compagnie Générale des Etablissements Michelin, 63000 Clermont-Ferrand (FR)
(72) Inventeur: GANDER, Sophie, F-63040 Clermont-Ferrand Cedex 9 (FR); SALIT, Anne-Frédérique, F-63040 Clermont-Ferrand Cedex 9 (FR); SEEBOTH, Nicolas, F-63040 Clermont-Ferrand Cedex 9 (FR)
(74) Mandataire: Gandon-Pain, Sylvie
(86) Numéro de dépôt international: PCT/EP2014/072845
(87) Numéro de publication internationale: WO 2015/059274

(56) Documents cités:
- EP-A1- 0 967 207
- WO-A1-2012/007442

## Description

Le domaine de la présente invention est celui des compositions de caoutchouc diénique renforcées par une charge et utilisables notamment pour la fabrication de pneumatiques pour véhicules. Elle se rapporte plus particulièrement aux bandes de roulement des pneumatiques à faible résistance au roulement.

Idéalement, une bande de roulement doit offrir au pneumatique un très bon niveau de comportement routier sur véhicule automobile. Ce niveau de comportement routier peut être apporté par l'utilisation dans la bande de roulement d'une composition de caoutchouc judicieusement choisie en raison de sa rigidité à cuit plutôt élevée. Pour augmenter la rigidité à cuit d'une composition de caoutchouc, il est connu par exemple d'augmenter le taux de charge ou de réduire le taux de plastifiant dans la composition de caoutchouc ou encore d'introduire des copolymères de styrène et de butadiène à fort taux de styrène dans la composition de caoutchouc. Mais certaines de ces solutions ont généralement pour inconvénient d'augmenter l'hystérèse de la composition de caoutchouc.

A l'inverse les compositions faiblement hystérétiques présentent le plus souvent une faible rigidité à cuit. Il peut s'avérer nécessaire de pallier cette baisse de rigidité à cuit pour assurer un comportement routier satisfaisant. Les Demanderesses ont par exemple décrit dans la demande de brevet WO 2011045131 une solution qui permet d'augmenter la rigidité à cuit d'une composition de caoutchouc faiblement hystérétique. Cette solution consiste à introduire du glycérol dans la composition de caoutchouc.

Les Demanderesses poursuivant leurs efforts pour obtenir une composition de caoutchouc rigide à cuit et faiblement hystérétique ont découvert que l'introduction d'un certain composé 1,3-dipolaire dans une composition de caoutchouc diénique renforcée par une charge permet d'atteindre ce but.

La présente invention a pour objet une composition de caoutchouc à base d'au moins un élastomère diénique, une charge renforçante et un composé 1,3-dipolaire répondant à la formule (I) :

Q-A-B (I)

dans laquelle :
Q comprend un dipôle contenant au moins et de préférence un atome d'azote,
A, de préférence divalent, est un atome ou un groupe d'atomes reliant Q à B,
B comprend un cycle imidazole répondant à la formule (II) : 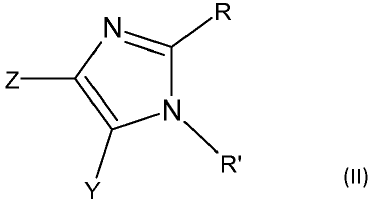 dans laquelle :
   3 des 4 symboles Z, Y, R et R' identiques ou différents représentent chacun un atome ou un groupe d'atomes, Z et Y pouvant former ensemble avec les atomes de carbone auxquels ils se rattachent un cycle,
   et le quatrième symbole Z, Y, R ou R' désigne un rattachement direct à A.

L'invention a aussi pour objet un procédé pour préparer une composition de caoutchouc à base d'au moins un élastomère diénique, un composé 1,3-dipolaire répondant à la formule (I) telle que définie ci-dessus, une charge renforçante et un système de réticulation, lequel procédé comprend les étapes suivantes :
- ajouter, au cours d'une première étape dite non productive, à l'élastomère diénique le composé 1,3-dipolaire, la charge renforçante, le cas échéant un agent de couplage en malaxant thermomécaniquement jusqu'à atteindre une température maximale comprise entre 130 et 200°C,
- refroidir l'ensemble à une température inférieure à 100°C,
- incorporer ensuite le système de réticulation,
- malaxer le tout jusqu'à une température maximale inférieure à 120°C.

L'invention a aussi pour objet une bande de roulement comprenant la composition de caoutchouc conforme à l'invention.

L'invention a encore pour objet un pneumatique comprenant la composition de caoutchouc conforme à l'invention, notamment dans sa bande de roulement.

### I. DESCRIPTION DETAILLEE DE L'INVENTION

Dans la présente description, sauf indication expresse différente, tous les pourcentages (%) indiqués sont des % en masse. L'abréviation "pce" signifie parties en poids pour cent parties d'élastomère (du total des élastomères si plusieurs élastomères sont présents). D'autre part, tout intervalle de valeurs désigné par l'expression "entre a et b" représente le domaine de valeurs supérieur à "a" et inférieur à "b" (c'est-à-dire bornes a et b exclues) tandis que tout intervalle de valeurs désigné par l'expression "de a à b" signifie le domaine de valeurs allant de "a" jusqu'à "b" (c'est-à-dire incluant les bornes strictes a et b).

Par l'expression composition "à base de", il faut entendre dans la présente description une composition comportant le mélange et/ou le produit de réaction in situ des différents constituants utilisés, certains de ces constituants de base (par exemple l'élastomère, la charge ou autre additif classiquement utilisé dans une composition de caoutchouc destinée à la fabrication de pneumatique) étant susceptibles de, ou destinés à réagir entre eux, au moins en partie, lors des différentes phases de fabrication de la composition destinée à la fabrication de pneumatique.

Une caractéristique essentielle de la composition de caoutchouc selon l'invention est de comprendre un élastomère diénique.

Par élastomère (ou indistinctement caoutchouc) "diénique", doit être compris de manière connue un (ou plusieurs) élastomère constitué au moins en partie (i.e., un homopolymère ou un copolymère) d'unités monomères diènes (monomères porteurs de deux doubles liaisons carbone-carbone, conjuguées ou non).

Ces élastomères diéniques peuvent être classés dans deux catégories : "essentiellement insaturés" ou "essentiellement saturés". On entend en général par "essentiellement insaturé", un élastomère diénique issu au moins en partie de monomères diènes conjugués, ayant un taux de motifs ou unités d'origine diénique (diènes conjugués) qui est supérieur à 15% (% en moles) ; c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques "essentiellement saturés" (taux de motifs d'origine diénique faible ou très faible, toujours inférieur à 15%). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

Ces définitions étant données, on entend plus particulièrement par élastomère diénique susceptible d'être utilisé dans les compositions conformes à l'invention:
(a) - tout homopolymère d'un monomère diène conjugué, notamment tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant de 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, l'homme du métier du pneumatique comprendra que la présente invention est de préférence mise en œuvre avec des élastomères diéniques essentiellement insaturés, en particulier du type (a) ou (b) ci-dessus.

Dans le cas de copolymères du type (b), ceux-ci contiennent de 20 à 99% en poids d'unités diéniques et de 1 à 80% en poids d'unités vinylaromatique.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène.

A titre de composés vinylaromatiques conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène.

Préférentiellement, l'élastomère diénique est un élastomère essentiellement insaturé choisi dans le groupe constitué par les polybutadiènes, les polyisoprènes, les copolymères de butadiène, les copolymères d'isoprène, et les mélanges de ces élastomères. A titre d'élastomère diénique convient tout particulièrement un polybutadiène (BR), un copolymère de butadiène et de styrène (SBR), un caoutchouc naturel (NR) ou un polyisoprène de synthèse (IR) présentant préférentiellement un taux molaire de liaison cis-1,4 supérieur à 90%.

La composition de caoutchouc conforme à l'invention comprend un composé 1,3-dipolaire. Le terme composé 1,3-dipolaire est compris selon la définition donnée par IUPAC.

Le composé 1,3-dipolaire répond à la formule (I) :

Q-A-B (I)

dans laquelle :
Q comprend un dipôle contenant au moins et de préférence un atome d'azote,
A, de préférence divalent, est un atome ou un groupe d'atomes reliant Q à B,
B comprend un cycle imidazole répondant à la formule (II) : 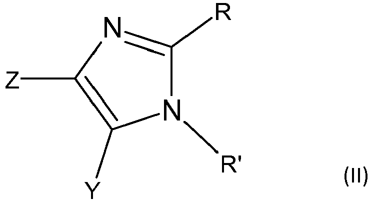 dans laquelle :
   3 des 4 symboles Z, Y, R et R' identiques ou différents représentent chacun un atome ou un groupe d'atomes, Z et Y pouvant former ensemble avec les atomes de carbone auxquels ils se rattachent un cycle (bien entendu lorsque ni Z, ni Y ne désigne le 4^{ème} symbole),
   et le seul quatrième symbole désigne un rattachement direct à A.

Selon une première variante de l'invention, R désigne un rattachement direct à A, auquel cas R est le 4^{ème} symbole.

Selon cette variante, R' peut être un atome d'hydrogène ou un groupe carboné pouvant contenir au moins un hétéroatome.

Selon un mode de réalisation préférentiel de cette variante, R' représente un groupe carboné contenant de 1 à 20 atomes de carbone, préférentiellement un groupe aliphatique, plus préférentiellement un groupe alkyle qui contient de préférence de 1 à 12 atomes de carbone.

Selon une seconde variante de l'invention, R' désigne un rattachement direct à A, auquel cas R' est le 4^{ème} symbole.

Selon la première ou la seconde variante, Z et Y peuvent être chacun un atome d'hydrogène.

Selon un autre mode de réalisation de la première variante ou de la seconde variante, Z et Y forment ensemble avec les atomes de carbone auxquels ils se rattachent un cycle. Le cycle formé par Z, Y et les atomes auxquels Z et Y se rattachent peut être substitué ou non et peut comporter au moins un hétéroatome. Z et Y peuvent former avec les deux atomes de carbone auxquels ils se rattachent un noyau aromatique. Dans ce cas, le cycle imidazole peut être un benzimidazole substitué ou non substitué.

Selon une troisième variante de l'invention, bien entendu lorsque Y et Z ne forment pas ensemble avec les atomes de carbone auxquels ils se rattachent un cycle, Y ou Z désigne un rattachement direct à A, auquel cas Y ou Z est le 4^{ème} symbole.

Selon un mode de réalisation particulier de la seconde ou de la troisième variante de l'invention, R représente un atome d'hydrogène ou un groupe carboné pouvant contenir au moins un hétéroatome.

Selon ce mode de réalisation particulier de la seconde variante ou de la troisième variante de l'invention, R peut être un groupe de 1 à 20 atomes de carbone, de préférence un groupe aliphatique, de manière plus préférentielle un groupe alkyle contenant de préférence de 1 à 12 atomes de carbone, de manière encore plus préférentielle un méthyle.

A peut être un groupe contenant jusqu'à 20 atomes de carbone, lequel groupe peut contenir au moins un hétéroatome. A peut être un groupe aliphatique ou aromatique.

Lorsque A est un groupe aliphatique, A contient préférentiellement de 1 à 20 atomes de carbone, plus préférentiellement de 1 à 12 atomes de carbone, encore plus préférentiellement de 1 à 6 atomes de carbone, tout particulièrement de 1 à 3 atomes de carbone. Lorsque A est un groupe aromatique, A contient préférentiellement de 6 à 20 atomes de carbone, plus préférentiellement de 6 à 12 atomes de carbone.

Comme groupe divalent A convient particulièrement un groupe alkylène contenant de 1 à 20 atomes de carbone, préférentiellement de 1 à 12 atomes de carbone, plus préférentiellement de 1 à 6 atomes de carbone, encore plus préférentiellement de 1 à 3 atomes de carbone. On peut citer comme groupe A divalent contenant de 1 à 3 atomes de carbone qui convient le groupe méthylène.

Comme groupe divalent A peut aussi convenir un groupe arylène contenant de préférence de 6 à 20 atomes de carbone, plus préférentiellement de 6 à 12 atomes de carbone. Conviennent tout particulièrement comme composés 1,3-dipolaires les composés choisis dans le groupe constitué par les oxydes de nitrile, les imines de nitrile et les nitrones, auquel cas Q contient un motif -C≡N→O, -C≡N→N- ou -C=N(→O)-.

Selon le mode de réalisation particulier de l'invention où Q comprend un motif -C≡N→O, Q de préférence comporte, de manière plus préférentielle représente le motif répondant à la formule (III) dans laquelle quatre des cinq symboles R1 à R5 identiques ou différents, sont chacun un atome ou un groupe d'atomes et le cinquième symbole désigne un rattachement direct à A, sachant que R1 et R5 sont tous les deux différents de H. Les quatre des cinq symboles R1 à R5 peuvent être des groupes aliphatiques ou aromatiques. Les groupes aliphatiques peuvent contenir de 1 à 20 atomes de carbone, préférentiellement de 1 à 12 atomes de carbone, plus préférentiellement de 1 à 6 atomes de carbone, encore plus préférentiellement de 1 à 3 atomes de carbone. Les groupes aromatiques peuvent contenir de 6 à 20 atomes de carbone, préférentiellement de 6 à 12 atomes de carbone.

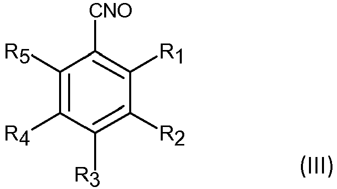

R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle.

Selon une variante de ce mode de réalisation particulier de l'invention, R1, R3 et R5 sont identiques. Selon cette variante où ils sont identiques, R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle.

De manière davantage préférentielle, le composé 1,3-dipolaire est le composé 2,4,6-triméthyl-3-((2-méthyl-1*H*-imidazol-1-yl)méthyl)benzonitrile oxyde répondant à la formule (IIIa) ou le composé 2,4,6-triéthyl-3-((2-méthyl-1*H*-imidazol-1-yl)méthyl)benzonitrile oxyde répondant à la formule (IIIb) :

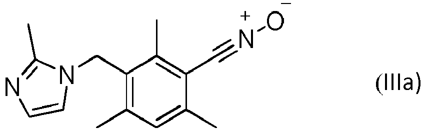

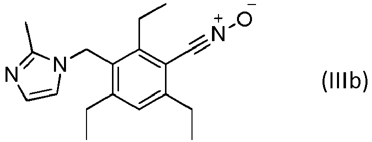

Selon le mode de réalisation particulier de l'invention où Q comprend un motif -C=N(→O)-, Q de préférence comporte, de manière plus préférentielle représente le motif répondant à la formule (IV) ou (V) 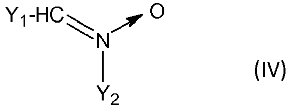 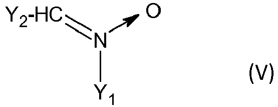 dans laquelle :
Y₁ est un groupe aliphatique, préférentiellement un groupe alkyle contenant de préférence 1 à 12 atomes de carbone, ou un groupe aromatique contenant de 6 à 20 atomes de carbone, préférentiellement un groupe alkylaryle, plus préférentiellement un groupe phényle ou tolyle,
et Y₂ , comportant un rattachement direct à A, est un groupe aliphatique, préférentiellement un groupe alkylène contenant de préférence 1 à 12 atomes de carbone, ou un groupe aromatique contenant préférentiellement de 6 à 20 atomes de carbone et comportant sur son noyau benzénique le rattachement direct à A.

Le rattachement direct du noyau benzénique de Y₂ à A revient à dire que A est un substituant du noyau benzénique de Y₂.

Selon ce mode de réalisation particulier de l'invention, le composé 1,3-dipolaire est le composé de formule (IVa), (IVb), (Va) ou (Vb)

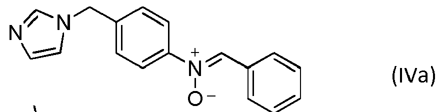

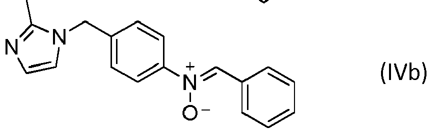

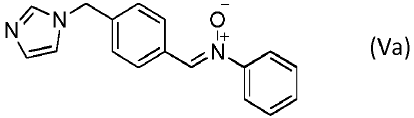

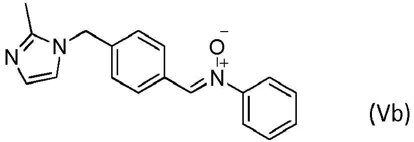

La quantité de composé 1,3-dipolaire introduite dans la composition de caoutchouc est exprimée en équivalent molaire de cycle imidazole. Par exemple, si le composé 1,3-dipolaire contient un seul cycle imidazole de formule (II) tel que définie précédemment, à une mole de composé 1,3-dipolaire correspond une mole de cycle imidazole. Si le composé 1,3-dipolaire contient deux cycles imidazole de formule (II) tel que définie précédemment, à une mole de composé 1,3-dipolaire correspond deux moles de cycle imidazole. Dans ce dernier cas l'utilisation du composé 1,3-dipolaire selon un équivalent molaire de cycle imidazole correspond à une demi-mole de composé 1,3-dipolaire.

Selon l'un quelconque mode de réalisation de l'invention, la quantité de composé 1,3-dipolaire dans la composition de caoutchouc est préférentiellement comprise entre 0 et 3 équivalents molaires, plus préférentiellement entre 0 et 2 équivalents molaires, encore plus préférentiellement entre 0 et 1 équivalent molaire, voire encore plus préférentiellement entre 0 et 0.7 équivalent molaire de cycle imidazole pour 100 moles d'unités monomères constituant l'élastomère diénique. Ces plages préférentielles permettent d'optimiser de façon plus fine le compromis entre la rigidité à cuit et l'hystérèse de la composition de caoutchouc selon son application, notamment dans un pneumatique. Pour chacune de ces plages préférentielles, la borne inférieure est de préférence d'au moins 0.1 équivalent molaire de composé 1,3-dipolaire.

La composition de caoutchouc conforme à l'invention comporte tout type de charge dite renforçante, connue pour ses capacités à renforcer une composition de caoutchouc utilisable pour la fabrication de pneumatiques, par exemple une charge organique telle que du noir de carbone, une charge inorganique renforçante telle que de la silice à laquelle est associé de manière connue un agent de couplage, ou encore un mélange de ces deux types de charge.

Une telle charge renforçante consiste typiquement en des nanoparticules dont la taille moyenne (en masse) est inférieure au micromètre, généralement inférieure à 500 nm, le plus souvent comprise entre 20 et 200 nm, en particulier et plus préférentiellement comprise entre 20 et 150 nm.

Comme noirs de carbone conviennent tous les noirs de carbone, notamment les noirs conventionnellement utilisés dans les pneumatiques ou leurs bandes de roulement (noirs dits de grade pneumatique). Parmi ces derniers, on citera plus particulièrement les noirs de carbone renforçants des séries 100, 200, 300, ou les noirs de série 500, 600 ou 700 (grades ASTM), comme par exemple les noirs N115, N134, N234, N326, N330, N339, N347, N375, N550, N683, N772). Ces noirs de carbone peuvent être utilisés à l'état isolé, tels que disponibles commercialement, ou sous tout autre forme, par exemple comme support de certains des additifs de caoutchouterie utilisés.

Par "charge inorganique renforçante", doit être entendu ici toute charge inorganique ou minérale, quelles que soient sa couleur et son origine (naturelle ou de synthèse), encore appelée charge "blanche", charge "claire" ou même charge "non-noire" par opposition au noir de carbone, capable de renforcer à elle seule, sans autre moyen qu'un agent de couplage intermédiaire, une composition de caoutchouc destinée à la fabrication de bandages pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

Comme charges inorganiques renforçantes conviennent notamment des charges minérales du type siliceuse, préférentiellement la silice (SiO₂). La silice utilisée peut être toute silice renforçante connue de l'homme du métier, notamment toute silice précipitée ou pyrogénée présentant une surface BET ainsi qu'une surface spécifique CTAB toutes deux inférieures à 450 m²/g, de préférence de 30 à 400 m²/g, notamment entre 60 et 300 m²/g. A titres de silices précipitées hautement dispersibles (dites "HDS"), on citera par exemple les silices « Ultrasil » 7000 et « Ultrasil » 7005 de la société Degussa, les silices « Zeosil » 1165MP, 1135MP et 1115MP de la société Rhodia, la silice « Hi-Sil » EZ150G de la société PPG, les silices « Zeopol » 8715, 8745 et 8755 de la Société Huber, les silices à haute surface spécifique telles que décrites dans la demande WO 03/016387.

Dans le présent exposé, la surface spécifique BET est déterminée de manière connue par adsorption de gaz à l'aide de la méthode de Brunauer-Emmett-Teller décrite dans "The Journal of the American Chemical Society" Vol. 60, page 309, février 1938, plus précisément selon la norme française NF ISO 9277 de décembre 1996 (méthode volumétrique multipoints (5 points) - gaz: azote - dégazage: 1heure à 160°C - domaine de pression relative *p*/*po :* 0.05 à 0.17). La surface spécifique CTAB est la surface externe déterminée selon la norme française NF T 45-007 de novembre 1987 (méthode B).

L'état physique sous lequel se présente la charge inorganique renforçante est indifférent, que ce soit sous forme de poudre, de microperles, de granulés, ou encore de billes. Bien entendu on entend également par charge inorganique renforçante des mélanges de différentes charges inorganiques renforçantes, en particulier de silices hautement dispersibles telles que décrites ci-dessus.

L'homme du métier comprendra qu'à titre de charge équivalente de la charge inorganique renforçante décrite dans le présent paragraphe, pourrait être utilisée une charge renforçante d'une autre nature, notamment organique telle que du noir de carbone, dès lors que cette charge renforçante serait recouverte d'une couche inorganique telle que silice, ou bien comporterait à sa surface des sites fonctionnels, notamment hydroxyles, nécessitant l'utilisation d'un agent de couplage pour établir la liaison entre la charge et l'élastomère. A titre d'exemple, on peut citer par exemple des noirs de carbone pour pneumatiques tels que décrits par exemple dans les documents brevet WO 96/37547, WO 99/28380.

Selon un mode de réalisation particulier de l'invention, la charge inorganique, préférentiellement une silice, représente plus de 50% en masse de la masse de la charge renforçante de la composition de caoutchouc. On dit alors que la charge inorganique renforçante est majoritaire.

Lorsqu'il est combiné à une charge inorganique renforçante majoritaire telle que la silice, le noir de carbone est utilisé de préférence à un taux inférieur à 20 pce, plus préférentiellement inférieur à 10 pce (par exemple entre 0.5 et 20 pce, notamment entre 2 et 10 pce). Dans les intervalles indiqués, on bénéficie des propriétés colorantes (agent de pigmentation noire) et anti-UV des noirs de carbone, sans pénaliser par ailleurs les performances typiques apportées par la charge inorganique renforçante.

Le taux de charge renforçante totale est compris préférentiellement entre 30 et 160 pce, plus préférentiellement entre 40 pce et 160 pce. En deçà de 30 pce, le renforcement de la composition de caoutchouc peut être insuffisant pour apporter un niveau de cohésion ou de résistance à l'usure adéquats du composant caoutchouteux du pneumatique comprenant cette composition. De manière encore plus préférentielle, le taux de charge renforçante totale est d'au moins 50 pce. Au-delà de 160 pce, il existe un risque d'augmentation de l'hystérèse et donc de la résistance au roulement des pneumatiques. Pour cette raison, le taux de charge renforçante totale est de préférence dans un domaine allant de 50 à 120 pce, notamment pour un usage dans une bande de roulement de pneumatique. L'une quelconque de ces plages de taux de charge renforçante totale s'applique à l'un quelconque des modes de réalisation de l'invention.

Pour coupler la charge inorganique renforçante à l'élastomère diénique, on utilise de manière bien connue un agent de couplage, notamment un silane, (ou agent de liaison) au moins bifonctionnel destiné à assurer une connexion suffisante, de nature chimique et/ou physique, entre la charge inorganique (surface de ses particules) et l'élastomère diénique. On utilise en particulier des organosilanes ou des polyorganosiloxanes au moins bifonctionnels.

On utilise notamment des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650).

Conviennent en particulier, sans que la définition ci-après soit limitative, des silanes polysulfurés répondant à la formule générale (V)

Z - A - Sₓ - A - Z (V)

dans laquelle :
- x est un entier de 2 à 8 (de préférence de 2 à 5) ;
- les symboles A, identiques ou différents, représentent un radical hydrocarboné divalent (de préférence un groupement alkylène en C₁-C₁₈ ou un groupement arylène en C₆-C₁₂, plus particulièrement un alkylène en C₁-C₁₀, notamment en C₁-C₄, en particulier le propylène) ;
- les symboles Z, identiques ou différents, répondent à l'une des trois formules ci-après: 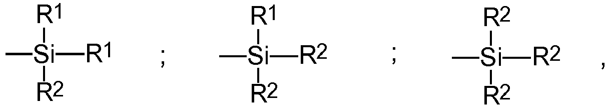
dans lesquelles:
- les radicaux R¹, substitués ou non substitués, identiques ou différents entre eux, représentent un groupe alkyle en C₁-C₁₈, cycloalkyle en C₅-C₁₈ ou aryle en C₆-C₁₈ (de préférence des groupes alkyle en C₁-C₆, cyclohexyle ou phényle, notamment des groupes alkyle en C₁-C₄, plus particulièrement le méthyle et/ou l'éthyle).
- les radicaux R², substitués ou non substitués, identiques ou différents entre eux, représentent un groupe alkoxyle en C₁-C₁₈ ou cycloalkoxyle en C₅-C₁₈ (de préférence un groupe choisi parmi alkoxyles en C₁-C₈ et cycloalkoxyles en C₅-C₈, plus préférentiellement encore un groupe choisi parmi alkoxyles en C₁-C₄, en particulier méthoxyle et éthoxyle).

Dans le cas d'un mélange d'alkoxysilanes polysulfurés répondant à la formule (I) ci-dessus, notamment des mélanges usuels disponibles commercialement, la valeur moyenne des "x" est un nombre fractionnaire de préférence compris entre 2 et 5, plus préférentiellement proche de 4. Mais l'invention peut être aussi avantageusement mise en œuvre par exemple avec des alkoxysilanes disulfurés (x = 2).

A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂)₃S]₂.

A titre d'agent de couplage autre qu'alkoxysilane polysulfuré, on citera notamment des POSS (polyorganosiloxanes) bifonctionnels ou encore des polysulfures d'hydroxysilane tels que décrits dans les demandes de brevets WO 02/30939 (ou US 6,774,255), WO 02/31041 (ou US 2004/051210) ou encore des silanes ou POSS porteurs de groupements fonctionnels azo-dicarbonyle, tels que décrits par exemple dans les demandes de brevets WO 2006/125532, WO 2006/125533, WO 2006/125534.

Bien entendu pourraient être également utilisés des mélanges des agents de couplage précédemment décrits, comme décrit notamment dans la demande WO 2006/125534 précitée.

La teneur en agent de couplage est avantageusement inférieure à 20 pce, étant entendu qu'il est en général souhaitable d'en utiliser le moins possible. Typiquement le taux d'agent de couplage représente de 0,5% à 15% en poids par rapport à la quantité de charge inorganique. Son taux est préférentiellement compris entre 0,5 et 12 pce, plus préférentiellement compris dans un domaine allant de 3 à 10 pce. Ce taux est aisément ajusté par l'homme du métier selon le taux de charge inorganique utilisé dans la composition.

La composition de caoutchouc conforme à l'invention peut également contenir, en complément des agents de couplage, des activateurs de couplage, des agents de recouvrement des charges inorganiques ou plus généralement des agents d'aide à la mise en œuvre susceptibles de manière connue, grâce à une amélioration de la dispersion de la charge dans la matrice de caoutchouc et à un abaissement de la viscosité des compositions, d'améliorer leur faculté de mise en œuvre à l'état cru.

La composition de caoutchouc conforme à l'invention peut comporter également tout ou partie des additifs usuels habituellement utilisés dans les compositions d'élastomères destinées à constituer des mélanges externes d'articles finis en caoutchouc tels que des pneumatiques, en particulier de bandes de roulement, comme par exemple des plastifiants ou des huiles d'extension, que ces derniers soient de nature aromatique ou non-aromatique, notamment des huiles très faiblement ou non aromatiques (e.g., huiles paraffiniques, naphténiques hydrogénées, huiles MES ou TDAE), des huiles végétales, en particulier les esters de glycérol comme les trioléates de glycérol, des résines plastifiantes hydrocarbonées présentant une haute Tg, de préférence supérieure à 30°C, telles que décrites par exemple dans les demandes WO 2005/087859, WO 2006/061064 et WO 2007/017060, des pigments, des agents de protection tels que cires anti-ozone, anti-ozonants chimiques, anti-oxydants, des agents anti-fatigue, des résines renforçantes (tels que résorcinol ou bismaléimide), des accepteurs (par exemple résine phénolique novolaque) ou des donneurs de méthylène (par exemple HMT ou H3M) tels que décrits par exemple dans la demande WO 02/10269, un système de réticulation, des accélérateurs ou retardateurs de vulcanisation, des activateurs de vulcanisation. Le système de réticulation est de préférence à base de soufre, mais il peut être également à base de donneurs de soufre, de peroxyde, de bismaléimides ou de leurs mélanges.

La composition de caoutchouc conforme à l'invention est fabriquée dans des mélangeurs appropriés, en utilisant deux phases de préparation successives bien connues de l'homme du métier : une première phase de travail ou malaxage thermomécanique (phase dite « non-productive ») à haute température, jusqu'à une température maximale comprise entre 130°C et 200°C, suivie d'une seconde phase de travail mécanique (phase dite « productive ») jusqu'à une plus basse température, typiquement inférieure à 110°C, par exemple entre 40°C et 100°C, phase de finition au cours de laquelle est incorporé le système de réticulation.

Le procédé pour préparer la composition de caoutchouc conforme à l'invention et à base d'au moins un système de réticulation comprend les étapes suivantes :
- ajouter au cours d'une première étape dite non productive à l'élastomère diénique le composé 1,3-dipolaire, la charge renforçante, le cas échéant l'agent de couplage, en malaxant thermomécaniquement jusqu'à atteindre une température maximale comprise entre 130 et 200°C,
- refroidir l'ensemble à une température inférieure à 100°C,
- incorporer ensuite le système de réticulation,
- malaxer le tout jusqu'à une température maximale inférieure à 120°C.

La quantité de composé 1,3-dipolaire ajoutée est préférentiellement comprise entre 0 et 3 équivalents molaires, plus préférentiellement entre 0 et 2 équivalents molaires, encore plus préférentiellement entre 0 et 1 équivalent molaire, voire encore plus préférentiellement entre 0 et 0.7 équivalent molaire de cycle imidazole pour 100 moles d'unités monomères constituant l'élastomère diénique. Pour chacune de ces plages préférentielles, la borne inférieure est de préférence d'au moins 0.1 équivalent molaire de composé 1,3-dipolaire.

Selon un mode de réalisation préférentiel de l'invention, le composé 1,3-dipolaire est incorporé à l'élastomère diénique avant l'introduction des autres constituants de la composition de caoutchouc. Le temps de contact entre l'élastomère diénique et le composé 1,3-dipolaire qui sont malaxés thermomécaniquement est ajusté en fonction des conditions du malaxage thermomécanique, notamment en fonction de la température. Plus la température du malaxage est élevée, plus ce temps de contact est court. Typiquement il est de 1 à 5 minutes pour une température de 100 à 130°C.

Selon ce mode de réalisation préférentiel de l'invention, on ajoute de préférence au moins un antioxydant à l'élastomère diénique avant son introduction dans un mélangeur, notamment à la fin de la synthèse de l'élastomère diénique comme cela se fait conventionnellement.

Après l'incorporation de tous les ingrédients de la composition de caoutchouc, la composition finale ainsi obtenue est ensuite calandrée, par exemple sous la forme d'une feuille ou d'une plaque, notamment pour une caractérisation au laboratoire, ou encore extrudée, pour former par exemple un profilé de caoutchouc utilisé comme composant caoutchouteux pour la confection du pneumatique.

Ainsi selon un mode de réalisation particulier de l'invention, la composition de caoutchouc conforme à l'invention, pouvant être soit à l'état cru (avant réticulation ou vulcanisation), soit à l'état cuit (après réticulation ou vulcanisation), est dans un pneumatique, notamment dans une bande de roulement de pneumatique.

Les caractéristiques précitées de la présente invention, ainsi que d'autres, seront mieux comprises à la lecture de la description suivante de plusieurs exemples de réalisation de l'invention, donnés à titre illustratif et non limitatif.

### II. EXEMPLES DE REALISATION DE L'INVENTION

### II.1-Mesures et tests utilisés :

### Analyse RMN :

L'analyse structurale ainsi que la détermination des puretés molaires des molécules de synthèses sont réalisées par une analyse RMN. Les spectres sont acquis sur un spectromètre Avance 3 400 MHz BRUKER équipé d'une sonde " large bande " BBFO-zgrad 5 mm. L'expérience RMN ¹H quantitative, utilise une séquence simple impulsion 30° et un délai de répétition de 3 secondes entre chacune des 64 acquisitions. Les échantillons sont solubilisés dans le Diméthylsulfoxide deutéré (DMSO). Ce solvant est également utilisé pour le signal de lock. La calibration est réalisée sur le signal des protons du DMSO deutéré à 2.44ppm par rapport à une référence TMS à 0ppm. Le spectre RMN ¹H couplé aux expériences 2D HSQC ¹H/13C et HMBC ¹H/¹³C permettent la détermination structurale des molécules (cf tableaux d'attributions). Les quantifications molaires sont réalisées à partir du spectre RMN 1D ¹H quantitatif.

### Essais de traction :

Ces essais de traction permettent de déterminer les contraintes d'élasticité. Sauf indication différente, ils sont effectués conformément à la norme française NF T 46-002 de septembre 1988. Un traitement des enregistrements de traction permet également de tracer la courbe de module en fonction de l'allongement. On mesure en première élongation le module sécant nominal calculé en se ramenant à la section initiale de l'éprouvette (ou contrainte apparente, en MPa) à 100% d'allongement noté MSA100. Toutes ces mesures de traction sont effectuées dans les conditions normales de température (23 ± 2°C) selon la norme NF T 46-002.

### Propriétés dynamiques :

Les propriétés dynamiques tan(δ)max sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99, ou selon les cas à une température différente (100°C). On effectue un balayage en amplitude de déformation de 0,1% à 100% (cycle aller), puis de 100% à 0,1% (cycle retour). Les résultats exploités sont le module complexe de cisaillement dynamique (G*) à 25% de déformation, le facteur de perte tan(δ) et l'écart de module (ΔG*) entre les valeurs à 0,1 et 100% de déformation (effet Payne). Pour le cycle retour, on indique la valeur maximale de tan(δ) observée, noté tan(δ)max.

### II.2-Synthèse du composé 1,3-dipolaire 2,4,6-triméthyl-3-((2-méthyl-1H-imidazol-1-yl)méthyl)benzonitrile oxyde :

Ce composé peut être préparé selon le schéma réactionnel suivant :

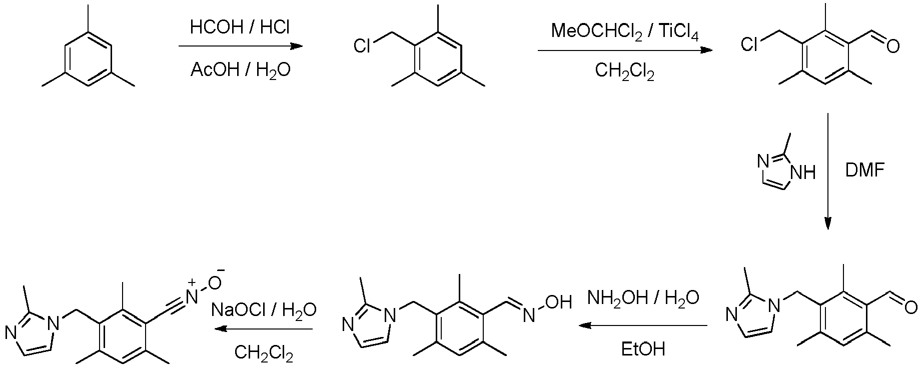

### 11.2-1-Synthèse du 2-(chlorométhyl)-1,3,5-triméthylbenzène :

Ce composé peut être obtenu selon une procédure décrite dans l'article suivant : Zenkevich, I. G.; Makarov, A. A.; Russian Journal of General Chemistry; vol. 77; nb. 4; (2007); p. 611-619 (Zhurnal Obshchei Khimii; vol. 77; nb. 4; (2007); p. 653 - 662)

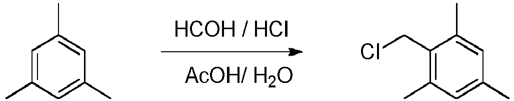

Un mélange de mésitylène (100,0 g, 0,832 mol), de para-formaldéhyde (26,2 g, 0,874 mol) et d'acide chlorhydrique (240 ml, 37 %, 2,906 mol) dans l'acide acétique (240 ml) est agité et chauffé très lentement (1,5 heures) jusqu'à 37°C. Après retour à température ambiante, le mélange est dilué par de l'eau (1,0 l) avec CH₂Cl₂ (200 ml), le produit est extrait par CH₂Cl₂ (4 fois par 50 mL). Les phases organiques sont rassemblées, puis lavées par l'eau (5 fois par 100 ml) et évaporées jusqu'à 11-12 mbar (Température du bain = 42°C). Une huile incolore (133,52 g, rendement 95 %) est obtenue. Après 15-18 heures à +4°C, l'huile a cristallisé. Les cristaux sont filtrés, lavés par de l'éther de pétrole refroidi à -18°C (40 ml), puis séchés pendant 3 à 5 heures sous pression atmosphérique à température ambiante. Un solide blanc (95,9 g, rendement 68 %) de point de fusion 39 °C est obtenu. La pureté molaire est supérieure à 96 % (RMN 1H).

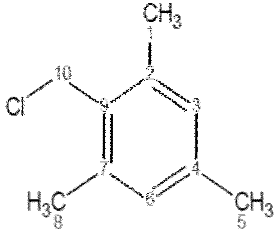

| **N°** | **δ ¹H (ppm)** | **δ ¹³C (ppm)** |
|---|---|---|
| 1-8 | 2,27 | 18,4 |
| 2-7 | - | 136,9 |
| 3-6 | 6,81 | 128,5 |
| 4 | - | 137,4 |
| 5 | 2,15 | 20,3 |
| 9 | - | 130,5 |
| 10 | 4,69 | 41,3 |

### II.2-2-Synthèse du 3-(chlorométhyl)-2,4,6-triméthylbenzaldéhyde:

Ce composé peut être obtenu selon une procédure décrite dans l'article suivant : Yakubov, A. P.; Tsyganov, D. V.; Belen'kii, L. I.; Krayushkin, M. M.; Bulletin of the Academy of Sciences of the USSR, Division of Chemical Science (English Translation); vol. 40; nb. 7.2; (1991); p. 1427 - 1432 (Izvestiya Akademii Nauk SSSR, Seriya Khimicheskaya; nb. 7; (1991); p. 1609 -1615)

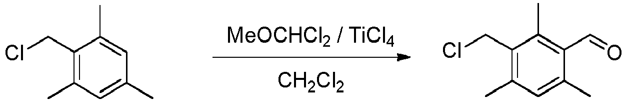

A une solution de TiCl₄ (90,0 g, 0,474 mol) dans le dichlorométhane (200 ml) à 17°C est ajoutée sous argon pendant 10-12 minutes une solution du 2-(chlorométhyl)-1,3,5-triméthylbenzène (20,0 g, 0,118 mol) et de dichlorométhylméthyl éther (27,26 g, 0,237 mol) dans le dichlorométhane (200 ml). Après agitation pendant 15-20 minutes à 17-20 °C, de l'eau (1000 ml) et de la glace (500 g) sont ajoutées au milieu réactionnel. Après 10-15 minutes d'agitation, la phase organique est séparée. La phase aqueuse est extraite par CH₂Cl₂ (3 fois par 75 ml). Les phases organiques rassemblées sont lavées par l'eau (4 fois par 100 ml) et évaporées sous pression réduite pour conduire à un solide (Température du bain = 28°C). Le produit cible (22,74 g) est obtenu avec un rendement de 97 %. Son point de fusion est de 58 °C. La pureté molaire estimée par RMN ¹H est de 95%mol.

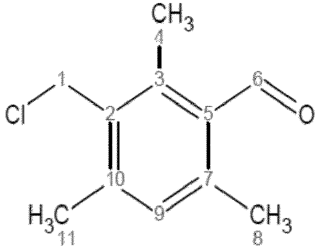

| **N°** | **δ ¹H (ppm)** | **δ ¹³C (ppm)** |
|---|---|---|
| 1 | 4,77 | 40,6 |
| 2 | - | 132,9 |
| 3 | - | 139,5 |
| 4 | 2,51 | 14,4 |
| 5 | - | 131,4 |
| 6 | 10,43 | 194,2 |
| 7 | - | 140,1 |
| 8 | 2,41 | 19,3 |
| 9 | 6,99 | 131,2 |
| 10 | - | 142,4 |
| 11 | 2,34 | 19,8 |

### 11.2-3-Synthèse du 2,4,6-triméthyl-3-((2-méthyl-1H-imidazol-1-yl)méthyl) benzaldéhyde :

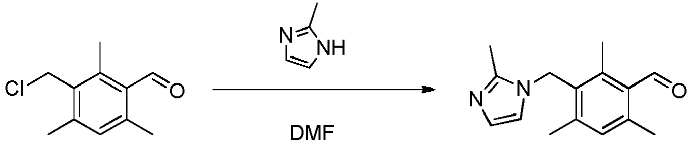

Un mélange de 3-(chlorométhyl)-2,4,6-triméthylbenzaldéhyde (10,0 g, 0,051 mol) et d'imidazole (10,44 g, 0,127 mol) dans le DMF (10 ml) est agité à 80°C pendant une heure. Après retour à 40-50°C, le mélange est dilué par l'eau (200ml), et agité pendant 10 minutes. Le précipité obtenu est filtré et lavé sur le filtre par l'eau (4 fois par 25 ml) puis séché à température ambiante. Un solide blanc (7,92 g, rendement 64 %) de point de fusion de 161 °C est obtenu. La pureté molaire est 91 % (RMN ¹H).

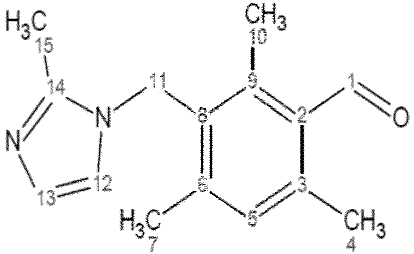

| **N°** | **δ ¹H (ppm)** | **δ ¹³C (ppm)** |
|---|---|---|
| 1 | 10,45 | 194,2 |
| 2 | - | 131,5 |
| 3 | - | 139,5 |
| 4 | 2,44 | 19,6 |
| 5 | 7,04 | 131,2 |
| 6 | - | 142,5 |
| 7 | 2,19 | 19,5 |
| 8 | - | 131 |
| 9 | - | 139,5 |
| 10 | 2,34 | 14,6 |
| 11 | 5,02 | 42,5 |
| 12 | 6,24 | 116,9 |
| 13 | 6,59 | 125,9 |
| 14 | - | 143,5 |
| 15 | 2,32 | 12,7 |

### II.2-4-Synthèse du 2,4,6-triméthyl-3-((2-méthyl-1H-imidazol-1-yl)méthyl)benzaldéhyde oxime:

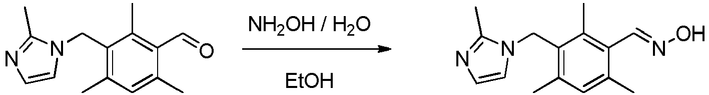

A une solution de 2,4,6-triméthyl-3-((2-méthyl-1H-imidazol-1-yl)méthyl) benzaldéhyde (20.3 g, 0,084mol) dans EtOH (110 ml) à 40°C, est ajoutée une solution aqueuse d'hydroxylamine (809 g, 0,134 mol, 50 % dans l'eau, Aldrich) dans EtOH (10 ml). Le milieu réactionnel est agité pendant 2,5 heures à une température de 50 à 55°C. Après retour à 23°C, le précipité obtenu est filtré et lavé deux fois sur le filtre par un mélange EtOH/H₂O (10 ml/15 ml) et séché pendant 15 à 20 heures sous pression atmosphérique à température ambiante. Un solide blanc (19.57 g, rendement 91 %) de point de fusion de 247°C est obtenu. La pureté molaire est supérieure à 87 % (RMN ¹H).

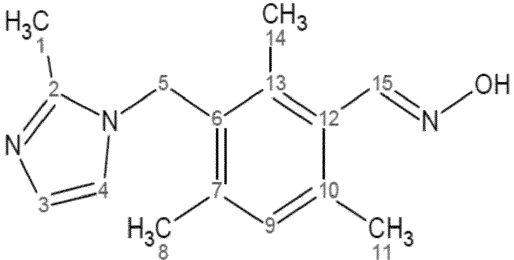

| **N°** | **δ ¹H (ppm)** | **δ ¹³C (ppm)** |
|---|---|---|
| 1 | 2,31 | 12,7 |
| 2 | - | 143,4 |
| 3 | 6,58 | 125,8 |
| 4 | 6,22 | 116,9 |
| 5 | 4,97 | 43,2 |
| 6 | - | 129,3 |
| 7 | - | 136,2 |
| 8 | 2,23 | 20,2 |
| 9 | 6,97 | 130 |
| 10 | - | 137,3 |
| 11 | 2,15 | 19,1 |
| 12 | - | 129,1 |
| 13 | - | 136,1 |
| 14 | 2,11 | 15,9 |
| 15 | 8,25 | 147,4 |
| OH | 11,11 | - |

### II.2-5-Synthèse du 2,4,6-triméthyl-3-((2-méthyl-1H-imidazol-1-yl)méthyl)benzonitrile oxyde :

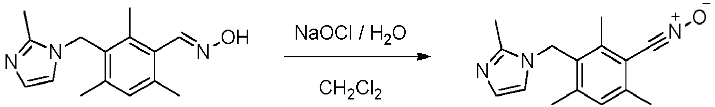

A un mélange de 2,4,6-triméthyl-3-((2-méthyl-1H-imidazol-1-yl)méthyl)benzaldéhyde oxime (8,80 g, 0,034 mol) dans CH₂Cl₂ (280 ml) à 6°C est ajoutée goutte à goutte une solution aqueuse de NaOCl (4 % de chlore actif, Aldrich, 49 ml) pendant 5 minutes. La température du milieu réactionnel est maintenue entre 6 et 8°C. Le milieu réactionnel est ensuite agité pendant 2 heures de 8°C à 21°C. La phase organique est séparée. La phase organique est lavée par l'eau (3 fois par 50 ml). Après concentration sous pression réduite (température du bain = 22-23°C, 220 mbar), de l'éther de pétrole (10 ml) est ajouté, le solvant est évaporé jusqu'à 8-10 ml, et la solution est maintenue à -18°C pendant 10-15 heures de façon à obtenir un précipité. Le précipité est filtré et lavé sur le filtre par le mélange de CH₂Cl₂ / éther de pétrole (2 ml / 6 ml) puis par l'éther de pétrole (2 fois 10 ml) et enfin séché pendant 10-15 heures sous pression atmosphérique à température ambiante. Un solide blanc (5,31 g, rendement 61 %) de point de fusion de 139 °C est obtenu.

La pureté molaire est supérieure à 95 % mol (RMN ¹H).

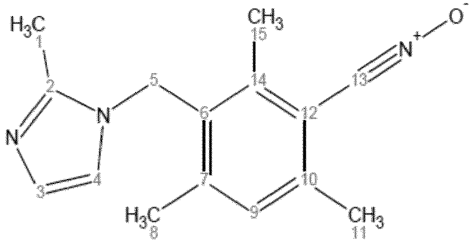

| **N°** | **δ ¹H (ppm)** | **δ ¹³C (ppm)** |
|---|---|---|
| 1 | 2,3 | 12,6 |
| 2 | - | 143,6 |
| | 6,59 | 126,1 |
| 4 | 6,27 | 117,1 |
| 5 | 4,99 | 43 |
| 6 | - | 130,6 |
| 7 | - | 140,7 |
| 8 | 2,16 | 19,2 |
| 9 | 7,12 | 129,9 |
| 10 | - | 141 |
| 11 | 2,34 | 20 |
| 12 | - | 112,1 |
| 13 | - | Nl |
| 14 | - | 140,8 |
| 15 | 2,28 | 17,7 |

### II.3-Synthèse du composé 1,3-dipolaire 2,4,6-triméthyl-3-((2-méthyl-1H-benzo[d]imidazol-1-yl)méthyl)benzonitrile oxide :

### 11.3-1-Synthèse du 2-(chlorométhyl)-1,3,5-triméthylbenzène :

La synthèse est à l'identique de celle décrite dans le paragraphe II.2-1.

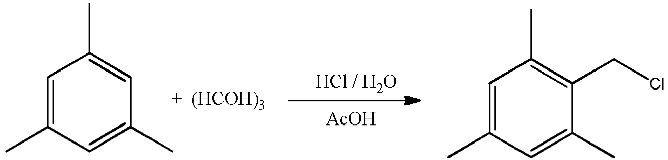

### II.3-2-Synthèse du 3-(chlorométhyl)-2,4,6-triméthylbenzaldéhyde:

La synthèse est à l'identique de celle décrite dans le paragraphe II.2-2.

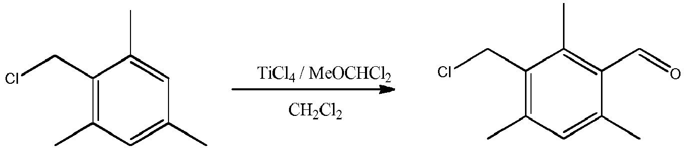

### II.3-3-Synthèse du 2,4,6-triméthyl-3-((2-méthyl-1H-benzo[d]imidazol-1-yl)méthyl)benzaldéhyde :

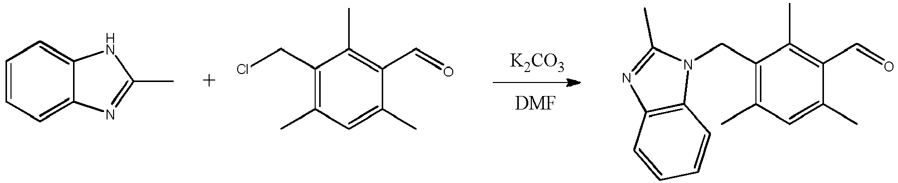

Un mélange d'aldéhyde (11,9 g, 60,5 mmol), de 2-méthylbenzimidazole (8,00 g, 60,5 mmol) et de carbonate de potassium (6,27 g, 45,4 mmol) dans le DMF (diméthylformamide, 15 ml) est agité pendant 1 heure à 80 °C et trois heures à 90 °C. Le mélange est ensuite dilué par l'eau (600ml). La phase organique est extraite par EtOAc (trois fois 150 ml) et lavée à l'eau (4 fois 75 ml). Les solvants sont évaporés sous pression réduite (36°C (T_{bain}) pour conduire à une huile brune. Celle-ci est cristallisée avec l'éther de pétrole 40/60 (15 ml) et l'éthylacétate (20 ml).
Un solide (11,70 g, 40,0 mmol, rendement 66 %) de point de fusion 118 °C est obtenu. La pureté molaire est 70 %, EtOAc - 5 % (RMN ¹H).

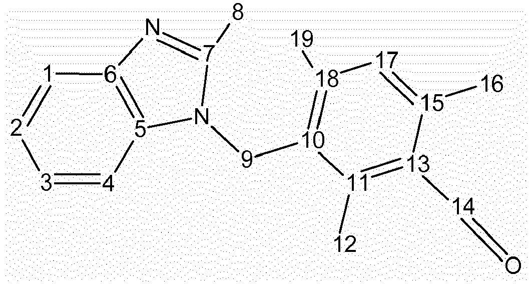

### Solvant : OMSO

| **N°** | **δ ₁H (ppm)** | **δ ₁₃C (ppm)** |
|---|---|---|
| 1 | 7.45 | 118.0 |
| 2 | 7.01 | 120.5 |
| 3 | 6.93 | 121.2 |
| 4 | 6.19 | 109.6 |
| 5 | / | 134.9 |
| 6 | / | 142.1 |
| 7 | / | 151.8 |
| 8 | 2.38 | 14.1 |
| 9 | 5.42 | 42.6 |
| 10 | / | ∼131 |
| 11 | / | 139.4 |
| 12 | 2.28 | 15.1 |
| 13 | / | 131.7 |
| 14 | 10.44 | 194.3 |
| 15 | / | 142.4 |
| 16 | ∼2.44 | 19.8 |
| 17 | 7.04 | 131.2 |
| 18 | / | ∼141.8 |
| 19 | 2.18 | 20,3 |

### II.3-4-Synthèse du 2,4,6-triméthyl-3-((2-méthyl-1H-benzo[d]imidazol-1-yl)méthyl)benzaldé-hyde oxime :

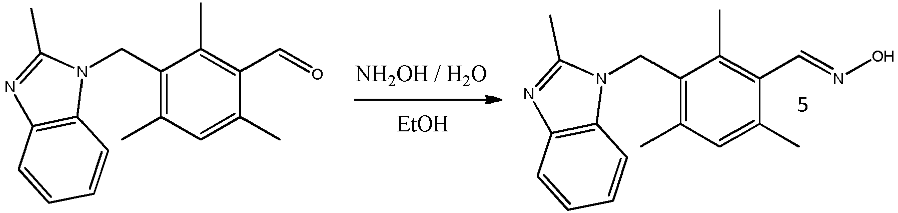

À une solution d'aldéhyde (11,5 g, 39,4 mmol) dans EtOH (80 ml) à 35 °C est ajoutée une solution d'hydroxylamine (6,14 g, 62,9 mmol, 50 % dans l'eau, Aldrich) dans EtOH (20 ml). Le milieu réactionnel est agité pendant 3,5 heures à 48-50°C. Le milieu réactionnel est ensuite refroidi jusqu'au 10-15 °C, le précipité obtenu est filtré et lavé sur le filtre par un mélange d'éthanol et d'eau (deux fois par mélange 5 ml et 10 ml) puis séché pendant 15-20 heures sous pression atmosphérique à température ambiante.
Un solide (7,95 g, 25,9 mmol, rendement 66 %) de point de fusion 248 °C est obtenu. La pureté molaire est supérieure à 80 % (RMN ¹H).

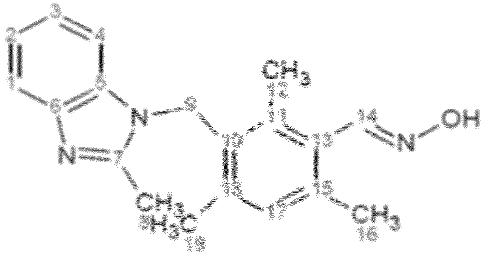

| **N°** | **δ ¹H (ppm)** | **δ ¹³C (ppm)** |
|---|---|---|
| 1 | 7,43 | 117,8 |
| 2 | 7,01 | 120,3 |
| 3 | 6,91 | 121 |
| 4 | 6,78 | 109,6 |
| 5 | / | 134,9 |
| 6 | / | 142 |
| 7 | / | 151,7 |
| 8 | 2,37 | 14 |
| 9 | 5,37 | 43,1 |
| 10/11/13/18 | / | entre 129,3 et 136,2 |
| 12 | 2,06 | 16,3 |
| 14 | 8.24 | 147,3 |
| 15 | / | 137,1 |
| 16 | 2,23 | 20,3 |
| 17 | 6,96 | 130,1 |
| 19 | 2,12 | 19,6 |

### II.3-5-Synthèse du 2,4,6-triméthyl-3-((2-méthyl-1H-benzo[d]imidazol-1-yl)méthyl)benzo-nitrile oxide :

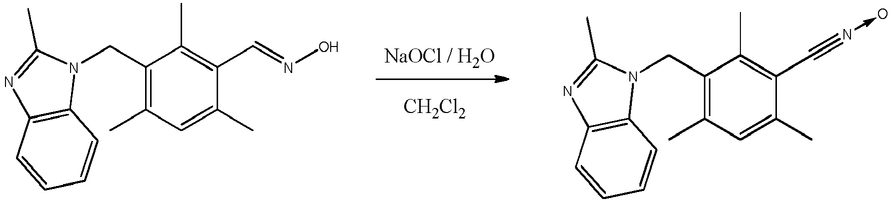

A une solution d'oxime (6,20 g, 20,2 mmol) dans le dichlorométhane (150 ml) refroidi jusqu'à 5°C est ajoutée goutte à goutte une solution aqueuse de NaOCl (6 % du chlore actif) (25,4 ml) pendant 6-8 minutes. Le milieu réactionnel est agité pendant 4,5 heures jusqu'à émulsion à 10°C. La phase organique est séparée et lavée par l'eau (3 fois par 25 ml). Après évaporation du solvant sous pression réduite (T_{bain} 22-23 ºC) jusqu'à cristallisation, de l'éther de pétrole (40/60) (10 ml) et du dichlorométhane (4 ml) sont ajoutés. La suspension est agitée pendant 10-15 minutes et le précipité est filtré, lavé sur le filtre par le mélange de CH₂Cl₂ / éther de pétrole (2 ml / 4 ml) et par l'éther de pétrole (40/60) (6 ml) et enfin séché pendant 10-15 heures sous pression atmosphérique à température ambiante.
Un solide blanc (4,85 g, 15,9 mmol, rendement 79 %) de point de fusion 142 °C est obtenu. La pureté molaire est supérieure à 71 % (RMN ¹H).
Le produit brut (4,4 g) est remis en solution dans l'acétone (100 ml), puis cette solution versée dans l'eau (500 ml), la suspension est agitée pendant 5-10 minutes. Le précipité est filtré et lavé sur le filtre par l'eau (200 ml), séché pendant 10-15 heures sous pression atmosphérique à température ambiante.
Un solide blanc (3,82 g, 12,6 mmol, rendement 62 %) de point de fusion 136,5-137,5 °C est obtenu avec une pureté de 94%mol en RMN ¹H.

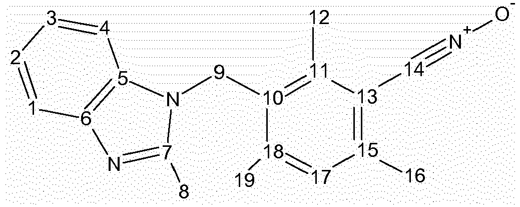

| **N°** | **δ ¹H (ppm)** | **δ ¹³C (ppm)** |
|---|---|---|
| 1 | 7.45 | 118.2 |
| 2 | 7.02 | 120.7 |
| 3 | 6.95 | 121.2 |
| 4 | 6.81 | 109.6 |
| 5 | / | 134.7 |
| 6 | / | 141.9 |
| 7 | / | 151.7 |
| 8 | 2.36 | 13.9 |
| 9 | 5.39 | 42.8 |
| 10 | / | 130.5 |
| 11 | / | entre 140.2 et 140.6 |
| 12 | 2.24 | 18.0 |
| 13 | / | 112.3 |
| 14 | / | Non Détecté |
| 15 | / | 140.9 |
| 16 | 2.34 | 19.9 |
| 17 | / | 130.2 |
| 18 | / | entre 140.2 et 140.6 |
| 19 | 2.1 | 19.7 |

Solvant: DMSO

### II.4-Préparation des compositions de caoutchouc :

On utilise les composés 1,3-dipolaires dont la synthèse est décrite ci-dessus.

On procède pour la fabrication de ces compositions de la manière suivante : on introduit dans un mélangeur interne (taux de remplissage final : environ 70% en volume), dont la température initiale de cuve est d'environ 110°C, l'élastomère, le cas échéant le composé 1,3-dipolaire qui est malaxé seul avec l'élastomère pendant environ 2 minutes à 110°C, puis la silice, l'agent de couplage, ainsi que les divers autres ingrédients à l'exception du système de vulcanisation. On conduit alors un travail thermomécanique (phase non-productive) en une étape, qui dure environ 5 min à 6 minutes, jusqu'à atteindre une température maximale de « tombée » de 160°C. On récupère le mélange ainsi obtenu, on le refroidit puis on incorpore du soufre et un accélérateur type sulfénamide sur un mélangeur (homo-finisseur) à 23°C, en mélangeant le tout (phase productive) pendant un temps approprié (par exemple entre 5 et 12 min).
Les compositions ainsi obtenues sont ensuite calandrées, soit sous forme de plaques (d'une épaisseur allant de 2 à 3 mm) ou fines feuilles de caoutchouc, pour la mesure de leurs propriétés physiques ou mécaniques, soit sous la forme de profilés directement utilisables, après découpage et/ou assemblage aux dimensions souhaitées, par exemple comme produits semi-finis pour pneumatiques, en particulier pour des bandes de roulement.
La réticulation est effectuée à 150°C. Le temps de réticulation appliqué, *t'_{c}*(90), est le temps nécessaire pour que le couple de la composition atteigne 90% du couple maximum de la composition. Les couples de la composition sont mesurés à 150°C avec un rhéomètre à chambre oscillante, selon la norme DIN 53529 - partie 3 (juin 1983). *t'_{c}*(90), est déterminé selon la norme NF T 43-015 pour chacune des compositions. D'une composition à une autre, il varie environ de 20 à 40 minutes.

### II-4.1 Exemple 1 :

Les formulations (en pce) des compositions A, B, C et D sont décrites dans le tableau I. Les compositions A et B à base de SBR et de silice diffèrent en ce que la composition B contient le composé 1,3-dipolaire dont la synthèse est décrite dans le paragraphe II-2. Les compositions C et D à base de polyisoprène et de silice diffèrent en ce que la composition D contient le composé 1,3-dipolaire.
Les compositions B et D sont conformes à l'invention. Les compositions A et C ne sont pas conformes à l'invention et sont les compositions témoin respectives des compositions B et D.

**Tableau I**

| Composition | A non conforme | B conforme | C non conforme | D conforme |
|---|---|---|---|---|
| SBR (1) | 100 | 100 | - | - |
| IR (2) | - | - | 100 | 100 |
| Composé 1,3-dipolaire (3) | - | 1.16 | - | 1.16 |
| Noir de carbone N234 | 3 | 3 | 3 | 3 |
| Silice (4) | 55 | 55 | 55 | 55 |
| Silane (5) | 5.5 | 5.5 | 5.5 | 5.5 |
| Antioxydant (6) | 1.5 | 1.5 | 1.5 | 1.5 |
| Antioxydant (7) | 1 | 1 | 1 | 1 |
| Cire anti-ozone | 1 | 1 | 1 | 1 |
| ZnO | 2.7 | 2.7 | 2.7 | 2.7 |
| Acide stéarique | 2.5 | 2.5 | 2.5 | 2.5 |
| Sulfénamide (8) | 1.8 | 1.8 | 1.8 | 1.8 |
| Soufre | 1.5 | 1.5 | 1.5 | 1.5 |

| | | | | |
|---|---|---|---|---|
| (1) SBR : SBR avec 25% de motif styrène et 56% de motif 1,2 de la partie butadiénique (2) IR : polyisoprène Natsyn 2200 (3) Composé 1,3-dipolaire dont la synthèse est décrite ci-dessus dans le paragraphe II.2 (4) Silice « Zeosil 1165 MP » de la société Rhodia (type HDS) (5) TESPT (« Si69 » de la société Degussa) (6) 2,2,4-triméthyl-1,2-dihydroquinoline (7) N-(1,3-diméthylbutyl)-N'-phényl-p-phénylènediamine, de la société Flexsys (8) N-cyclohexyl-2-benzothiazol-sulfénamide (« Santocure CBS » de la société Flexsys) | | | | |

Les résultats sont consignés dans le tableau (II) ci-après.

**Tableau (II)**

| **Composition** | A | B | C | D |
|---|---|---|---|---|
| **Propriétés à cuit** | | | | |
| MSA100 à 23°C | 2.83 | 3.47 | 2.31 | 2.61 |
| tanδ max à 23°C | 0.28 | 0.19 | 0.21 | 0.05 |
| ΔG* à 23°C | 4 | 0.23 | 5.36 | 0.35 |
| G*à 100°C | 1.61 | 1.78 | 1.59 | 1.50 |
| tanδ max à 100°C | 0.13 | 0.08 | 0.12 | 0.05 |

Les compositions B et D présentent à 23°C un module MSA100 à 23°C qui est bien supérieur à celui des compositions témoin respectives A et C. Cette hausse de rigidité à cuit est obtenue bien qu'une diminution très significative de l'hystérèse à 23°C est également observée pour B et D en comparaison à leur témoin respectif A et C. L'augmentation de la rigidité à cuit est d'autant plus remarquable que la baisse d'hystérèse est très forte, puisque la valeur de tanδ max à 23°C diminue de 32% pour la matrice SBR et de 76% pour la matrice IR et celle de ΔG* à 23°C diminue de 94% pour la matrice SBR et de 93% pour la matrice IR.
Un bon comportement routier d'un pneumatique étant généralement associé à une forte rigidité à cuit de la composition qui constitue sa bande de roulement, ce résultat augure un bon comportement routier d'un pneumatique ayant une bande de roulement comportant une composition B ou D.

Par ailleurs il est observé que comparativement à leur témoin respectif, les compositions selon l'invention B et D conservent un niveau de rigidité à cuit à 100°C comparable, voire même meilleur pour la composition B. Ces résultats présagent d'une polyvalence en température de la composition de caoutchouc conforme à l'invention. En effet on peut attendre qu'une bande de roulement contenant la composition B ou D permette au pneumatique d'avoir un comportement routier au moins tout aussi bon que ne le ferait la composition témoin A ou C, lors de conditions de roulage plus extrêmes, notamment pour des pneumatiques de voiture sport roulant à haute vitesse.

### II-4.2 Exemple 2 :

Les formulations (en pce) des compositions E et F sont décrites dans le tableau (III). Les compositions E et F à base de polyisoprène (IR) et de silice diffèrent en ce que la composition F contient le composé 1,3-dipolaire dont la synthèse est décrite dans le paragraphe II-3. Les compositions C et D à base de polyisoprène et de silice diffèrent en ce que la composition D contient le composé 1,3-dipolaire.

**Tableau III**

| Composition | E non conforme | F conforme |
|---|---|---|
| IR (1) | 100 | 100 |
| Composé 1,3-dipolaire (2) | - | 1.16 |
| Noir de carbone N234 | 3 | 3 |
| Silice (3) | 55 | 55 |
| Silane (4) | 5.5 | 5.5 |
| Antioxydant (5) | 1 | 1 |
| Antioxydant (6) | 1.5 | 1.5 |
| Cire anti-ozone | 1 | 1 |
| ZnO | 2.7 | 2.7 |
| Acide stéarique | 2.5 | 2.5 |
| Sulfénamide (7) | 1.8 | 1.8 |
| Soufre | 1.5 | 1.5 |

| | | |
|---|---|---|
| (1) IR : polyisoprène contenant 98% en poids de motif ,4-cis (2) Composé 1,3-dipolaire dont la synthèse est décrite ci-dessus dans le paragraphe II.3 (3) Silice « Zeosil 1165 MP » de la société Rhodia (type HDS) (4) TESPT (« Si69 » de la société Degussa) (5) 2,2,4-triméthyl-l,2-dihydroquinoline (6) N-(1,3-diméthylbutyl)-N'-phényl-p-phénylènediamine, de la société Flexsys (7) N-cyclohexyl-2-benzothiazol-sulfénamide (« Santocure CBS » de la société Flexsys) | | |

Les résultats sont consignés dans le tableau (IV) ci-après.

**Tableau (IV)**

| **Composition** | E | F |
|---|---|---|
| **Propriétés à cuit** | | |
| MSA100 à 23°C | 1.84 | 2.55 |
| tanδ max à 23°C | 0.23 | 0.07 |
| ΔG* à 23°C | 3.27 | 0.35 |
| G* à 100°C | 1.35 | 1.25 |
| tanδ max à 100°C | 0.13 | 0.05 |

La composition F présente à 23°C un module MSA100 à 23°C qui est bien supérieur à celui de la composition témoin E. Cette hausse de rigidité à cuit est obtenue bien qu'une diminution très significative de l'hystérèse à 23°C soit également observée pour F en comparaison au témoin E. L'augmentation de la rigidité à cuit est d'autant plus remarquable que la baisse d'hystérèse est très forte.

### II-4.3 Exemple 3 :

Les formulations (en pce) des compositions G, GT, H et HT sont décrites dans le tableau V. Les compositions G et H diffèrent de leur composition témoin respective GT et HT en ce qu'elles contiennent le composé 1,3-dipolaire dont la synthèse est décrite dans le paragraphe II.2. Les silanes sont introduits dans la composition à iso taux molaire de silicium : le silane disulfure ne libérant pas de soufre contrairement au silane TESPT, le taux de soufre soluble a été augmenté de 80% dans les compositions H et HT pour être à iso taux de soufre des autres compositions G et GT.

**Tableau (V)**

| Composition | GT non conforme | G conforme | HT non conforme | H conforme |
|---|---|---|---|---|
| SBR (1) | 100 | 100 | 100 | 100 |
| Noir de carbone (2) | 4 | 4 | 4 | 4 |
| Silice (3) | 84 | 84 | 84 | 84 |
| Silane (4) | 6.7 | 6.7 | - | - |
| Silane (5) | - | - | 5.93 | 5.93 |
| Composé 1,3-dipolaire (6) | - | 1.88 | - | 1.88 |
| Antioxydant (7) | 1.9 | 1.9 | 1.9 | 1.9 |
| Huile (8) | 4.8 | 4.8 | 4.8 | 4.8 |
| Cire anti-ozone | 1.5 | 1.5 | 1.5 | 1.5 |
| Résine (9) | 20 | 20 | 20 | 20 |
| ZnO | 2.5 | 2.5 | 2.5 | 2.5 |
| Acide stéarique | 2.5 | 2.5 | 2.5 | 2.5 |
| Soufre | 1.2 | 1.2 | 2.2 | 2.2 |
| Sulfénamide (10) | 1.9 | 1.9 | 1.9 | 1.9 |

| | | | | |
|---|---|---|---|---|
| (1) SBR : SBR avec 26% de motif styrène et 25% de motif 1,2 de la partie butadiénique (2) N234 (3) Silice « Zeosil 1165 MP » de la société Rhodia (type HDS) (4) TESPT (« Si69 » de la société Degussa) (5) « Si 75® » de la société Evonik (6) Composé 1,3-dipolaire dont la synthèse est décrite ci-dessus dans le paragraphe II.2 (7) N-(1,3-diméthylbutyl)-N'-phényl-p-phénylènediamine, de la société Flexsys (8) Huile MES ("Catenex SNR" de Shell) (9) Résine C5/C9 ECR-373 de la société Exxon (10) N-cyclohexyl-2-benzothiazol-sulfénamide (« Santocure CBS » de la société Flexsys) | | | | |

Les résultats sont consignés dans le tableau (VI) ci-après.

**Tableau (VI)**

| **Composition** | GT non conforme | G conforme | HT non conforme | H conforme |
|---|---|---|---|---|
| **Propriétés à cuit** | | | | |
| MSA100 à 23°C | 2.81 | 3.99 | 3.21 | 4.44 |
| MSA300 à 23°C | 3.15 | 5.05 | 3.69 | 4.99 |
| tanδ max à 23°C | 0.27 | 0.17 | 0.25 | 0.16 |
| ΔG* à 23°C | 7.14 | 1.59 | 9.11 | 1.7 |
| G* à 100°C | 1.73 | 1.87 | 2.09 | 1.99 |
| tanδ max à 100°C | 0.22 | 0.12 | 0.21 | 0.12 |

Les compositions conformes à l'invention présentent un compromis rigidité hystérèse très amélioré en comparaison à leur témoin respectif.

## Revendications

1. Composition de caoutchouc à base d'au moins un élastomère diénique, une charge renforçante et un composé 1,3-dipolaire répondant à la formule (I) :
Q-A-B (I)
dans laquelle :
Q comprend un dipôle contenant au moins et de préférence un atome d'azote,
A, de préférence divalent, est un atome ou un groupe d'atomes reliant Q à B,
B comprend un cycle répondant à la formule (II) 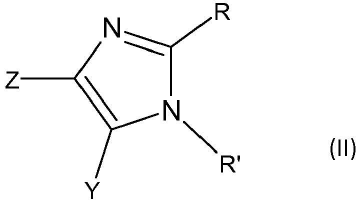 dans laquelle :
3 des 4 symboles Z, Y, R et R' identiques ou différents représentent chacun un atome ou un groupe d'atomes, Z et Y pouvant former ensemble avec les atomes de carbone auxquels ils se rattachent un cycle,
et le quatrième symbole Z, Y, R ou R' désigne un rattachement direct à A.

2. Composition de caoutchouc selon la revendication 1 dans laquelle R' désigne un rattachement direct à A.

3. Composition de caoutchouc selon la revendication 2 dans laquelle Z et Y sont chacun un atome d'hydrogène ou forment ensemble avec les atomes de carbone auxquels ils se rattachent un cycle, de préférence aromatique.

4. Composition de caoutchouc selon l'une quelconque des revendications 2 à 3 dans laquelle R représente un atome d'hydrogène ou un groupe carboné pouvant contenir au moins un hétéroatome et contenant de préférence de 1 à 20 atomes de carbone.

5. Composition de caoutchouc selon l'une quelconque des revendications 1 à 4 dans laquelle A est un groupe contenant jusqu'à 20 atomes de carbone et pouvant contenir au moins un hétéroatome.

6. Composition de caoutchouc selon l'une quelconque des revendications 1 à 5 dans laquelle le composé 1,3-dipolaire est choisi dans le groupe constitué par les oxydes de nitrile, les imines de nitrile et les nitrones.

7. Composition de caoutchouc selon la revendication 6 dans laquelle Q contient un motif - C≡N→O.

8. Composition de caoutchouc selon la revendication 7 dans laquelle Q comporte le motif répondant à la formule (III) : 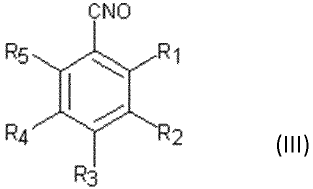 dans laquelle :
quatre des cinq symboles R1 à R5 identiques ou différents, sont chacun un atome ou un groupe d'atomes, préférentiellement un groupe aliphatique ou un groupe aromatique et le cinquième symbole désigne un rattachement direct à A, sachant que R1 et R5 sont tous les deux différents de H.

9. Composition de caoutchouc selon la revendication 8 dans laquelle R1, R3 et R5 sont chacun un groupe alkyle de 1 à 6 atomes de carbone, préférentiellement de 1 à 3 atomes de carbone.

10. Composition de caoutchouc selon la revendication 6 dans laquelle Q contient un motif - C=N(→O)-.

11. Composition de caoutchouc selon la revendication 10 dans laquelle Q comporte le motif répondant à la formule (IV) ou (V) 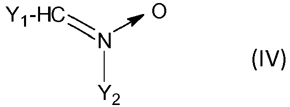 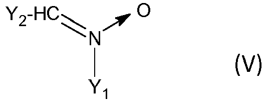 dans laquelle :
Y₁ est un groupe aliphatique, préférentiellement un groupe alkyle contenant de préférence 1 à 12 atomes de carbone, ou un groupe aromatique contenant de 6 à 20 atomes de carbone, préférentiellement un groupe alkylaryle, plus préférentiellement un groupe phényle ou tolyle,
Y₂, comportant un rattachement direct à A, est un groupe aliphatique, préférentiellement un groupe alkylène contenant de préférence 1 à 12 atomes de carbone, ou un groupe aromatique contenant préférentiellement de 6 à 20 atomes de carbone et comportant sur son noyau benzénique le rattachement direct à A.

12. Composition de caoutchouc selon l'une quelconque des revendications 1 à 11 dans laquelle la quantité de composé 1,3-dipolaire est comprise entre 0 et 3 équivalents molaires, préférentiellement entre 0 et 2 équivalents molaires, plus préférentiellement entre 0 et 1 équivalent molaire, encore plus préférentiellement entre 0 et 0.7 équivalent molaire de cycle imidazole pour 100 moles d'unités monomères constituant l'élastomère diénique.

13. Composition de caoutchouc selon l'une quelconque des revendications 1 à 12 dans laquelle l'élastomère diénique est un élastomère essentiellement insaturé choisi dans le groupe constitué par les polybutadiènes, les polyisoprènes, les copolymères de butadiène, les copolymères d'isoprène et les mélanges de ces élastomères.

14. Composition de caoutchouc selon l'une quelconque des revendications 1 à 13 dans laquelle la charge renforçante comprend une charge organique, de préférence un noir de carbone, ou une charge inorganique renforçante, de préférence une silice.

15. Procédé pour préparer une composition de caoutchouc à base d'au moins un élastomère diénique, un composé 1,3-dipolaire, une charge renforçante et un système de réticulation, lequel procédé comprend les étapes suivantes :
- ajouter au cours d'une première étape dite non productive à l'élastomère diénique le composé 1,3-dipolaire, la charge renforçante, le cas échéant un agent de couplage en malaxant thermomécaniquement jusqu'à atteindre une température maximale comprise entre 130 et 200°C,
- refroidir l'ensemble à une température inférieure à 100°C,
- incorporer ensuite le système de réticulation,
- malaxer le tout jusqu'à une température maximale inférieure à 120°C,
lequel composé 1,3-dipolaire est tel que défini à la revendication 1.

16. Bande de roulement qui comprend une composition de caoutchouc selon l'une quelconque des revendications 1 à 14.

17. Pneumatique qui comprend une composition de caoutchouc selon l'une quelconque des revendications 1 à 14, préférentiellement dans sa bande de roulement.

## Patentansprüche

1. Kautschukzusammensetzung auf Basis von mindestens einem Dienelastomer, einem Verstärkungsfüllstoff und einer 1,3-dipolaren Verbindung, die Formel (I) entspricht:
Q-A-B (I)
in der:
Q einen Dipol umfasst, der mindestens und vorzugsweise ein Stickstoffatom enthält,
A, vorzugsweise zweiwertig, ein Atom oder eine Gruppe von Atomen ist, das bzw. die Q mit B verbindet,
B einen Ring umfasst, der Formel (II) entspricht 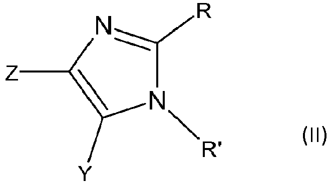 in der:
3 der 4 identischen oder unterschiedlichen Symbole Z, Y, R und R' jeweils ein Atom oder eine Gruppe von Atomen darstellen, wobei Z und Y gemeinsam mit den Kohlenstoffatomen, an die sie gebunden sind, einen Ring bilden können,
und das vierte Symbol Z, Y, R oder R' eine direkte Verbindung mit A angibt.

2. Kautschukzusammensetzung nach Anspruch 1, wobei R' eine direkte Verbindung mit A angibt.

3. Kautschukzusammensetzung nach Anspruch 2, wobei Z und Y jeweils ein Wasserstoffatom sind oder gemeinsam mit den Kohlenstoffatomen, mit denen sie verbunden sind, einen vorzugsweise aromatischen Ring bilden.

4. Kautschukzusammensetzung nach Anspruch 2 oder 3, wobei R ein Wasserstoffatom oder eine kohlenstoffhaltige Gruppe darstellt, die mindestens ein Heteroatom enthalten kann und vorzugsweise 1 bis 20 Kohlenstoffatome enthält.

5. Kautschukzusammensetzung nach einem der Ansprüche 1 bis 4, wobei A eine Gruppe ist, die bis zu 20 Kohlenstoffatome enthält und die mindestens ein Heteroatom enthalten kann.

6. Kautschukzusammensetzung nach einem der Ansprüche 1 bis 5, wobei die 1,3-dipolare Verbindung aus der Gruppe ausgewählt ist, die aus Nitriloxiden, Nitriliminen und Nitronen besteht.

7. Kautschukzusammensetzung nach Anspruch 6, wobei Q eine Einheit -C≡N→O enthält.

8. Kautschukzusammensetzung nach Anspruch 7, wobei Q die Einheit aufweist, die Formel (III): 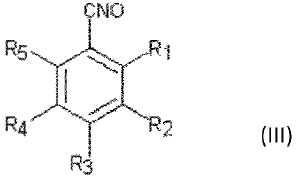 Entspricht, in der:
vier der fünf identischen oder unterschiedlichen Symbole R1 bis R5 jeweils ein Atom oder eine Gruppe von Atomen sind, bevorzugt eine aliphatische Gruppe oder eine aromatische Gruppe, und das fünfte Symbol eine direkte Verbindung mit A angibt, wobei sowohl R1 als auch R5 nicht H sind.

9. Kautschukzusammensetzung nach Anspruch 8, wobei R1, R3 und R5 jeweils eine Alkylgruppe mit 1 bis 6 Kohlenstoffatomen, vorzugsweise 1 bis 3 Kohlenstoffatomen sind.

10. Kautschukzusammensetzung nach Anspruch 6, wobei Q eine Einheit -C=N(→O)- enthält.

11. Kautschukzusammensetzung nach Anspruch 10, wobei Q die Einheit aufweist, die Formel (IV) oder (V) 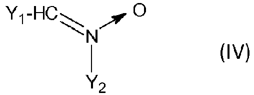 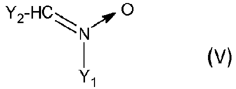 entspricht, in der:
Y₁ eine aliphatische Gruppe ist, vorzugsweise eine Alkylgruppe, die vorzugsweise 1 bis 12 Kohlenstoffatome enthält, oder eine aromatische Gruppe, die 6 bis 20 Kohlenstoffatome enthält, bevorzugt eine Alkylarylgruppe, bevorzugter eine Phenyl- oder Benzylgruppe,
Y₂, das eine direkte Verbindung mit A aufweist, eine aliphatische Gruppe ist, vorzugsweise eine Alkylengruppe, die vorzugsweise 1 bis 12 Kohlenstoffatome enthält, oder eine aromatische Gruppe, die bevorzugt 6 bis 20 Kohlenstoffatome enthält und an ihrem Benzolkern die direkte Verbindung mit A aufweist.

12. Kautschukzusammensetzung nach einem der Ansprüche 1 bis 11, wobei die Menge der 1,3-dipolaren Verbindung zwischen 0 und 3 Moläquivalenten, bevorzugt zwischen 0 und 2 Moläquivalenten, bevorzugter zwischen 0 und 1 Moläquivalent, noch bevorzugter zwischen 0 und 0,7 Moläquivalenten Imidazolring auf 100 Mol Monomereinheiten liegt, die das Dienelastomer bilden.

13. Kautschukzusammensetzung nach einem der Ansprüche 1 bis 12, wobei das Dienelastomer ein im Wesentlichen ungesättigtes Elastomer ist, das aus der Gruppe ausgewählt ist bestehend aus Polybutadienen, Polyisoprenen, Butadien-Copolymeren, Isopren-Copolymeren und Mischungen aus diesen Elastomeren.

14. Kautschukzusammensetzung nach einem der Ansprüche 1 bis 13, wobei der Verstärkungsfüllstoff einen organischen Füllstoff, vorzugsweise einen Industrieruß, oder einen anorganischen Verstärkungsfüllstoff, vorzugsweise ein Siliciumdioxid, umfasst.

15. Verfahren zur Herstellung einer Kautschukzusammensetzung auf Basis von mindestens einem Dienelastomer, einer dipolaren 1,3-Verbindung, einem Verstärkungsfüllstoff und einem Vernetzungssystem, wobei das Verfahren folgende Schritte umfasst:
- in einem ersten, sogenannten unproduktiven Schritt Zugeben der dipolaren 1,3-Verbindung, des Verstärkungsfüllstoffs und gegebenenfalls eines Kopplungsmittels zu dem Dienelastomer, durch thermomechanisches Mischen, bis eine Höchsttemperatur zwischen 130 und 200°C erreicht ist,
- Abkühlen der gesamten Mischung auf eine Temperatur unter 100°C,
- anschließend Einbringen des Vernetzungssystems,
- Vermischen sämtlicher Bestandteile bis auf eine Höchsttemperatur unter 120°C,
wobei die 1,3-dipolare Verbindung nach Anspruch 1 definiert ist.

16. Laufstreifen, der eine Kautschukzusammensetzung nach einem der Ansprüche 1 bis 14 umfasst.

17. Reifen, der eine Kautschukzusammensetzung nach einem der Ansprüche 1 bis 14 umfasst, vorzugsweise in seinem Laufstreifen.

## Claims

1. Rubber composition based on at least one diene elastomer, a reinforcing filler and a 1,3-dipolar compound corresponding to the formula (I):
Q-A-B (I)
in which:
Q comprises a dipole containing at least and preferably one nitrogen atom,
A, which is preferably divalent, is an atom or a group of atoms connecting Q to B,
B comprises a ring corresponding to the formula (II): 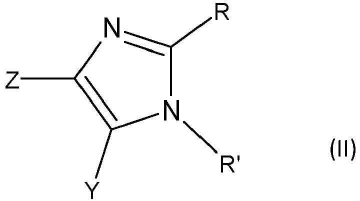
in which:
three of the four symbols Z, Y, R and R', which are identical or different, each represent an atom or a group of atoms, it being possible for Z and Y to form, together with the carbon atoms to which they are attached, a ring,
and the fourth symbol Z, Y, R or R' denotes a direct attachment to A.

2. Rubber composition according to Claim 1, in which R' denotes a direct attachment to A.

3. Rubber composition according to Claim 2, in which Z and Y are each a hydrogen atom or form, together with the carbon atoms to which they are attached, a ring, preferably an aromatic ring.

4. Rubber composition according to any one of Claims 2 to 3 in which R represents a hydrogen atom or a carbon-based group which can contain at least one heteroatom and preferably containing from 1 to 20 carbon atoms.

5. Rubber composition according to any one of Claims 1 to 4, in which A is a group containing up to 20 carbon atoms and which can contain at least one heteroatom.

6. Rubber composition according to any one of Claims 1 to 5, in which the 1,3-dipolar compound is selected from the group consisting of nitrile oxides, nitrile imines and nitrones.

7. Rubber composition according to Claim 6, in which Q contains a -C≡N→O unit.

8. Rubber composition according to Claim 16, in which Q comprises the unit corresponding to the formula (III): 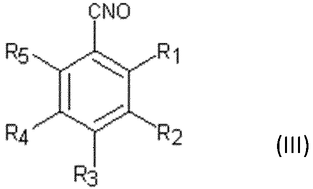 in which:
four of the five symbols R₁ to R₅, which are identical or different, are each an atom or a group of atoms, preferably an aliphatic group or an aromatic group, and the fifth symbol denotes a direct attachment to A, it being known that R₁ and R₅ are both other than H.

9. Rubber composition according to Claim 8, in which R₁, R₃ and R₅ are each an alkyl group of 1 to 6 carbon atoms, preferably of 1 to 3 carbon atoms.

10. Rubber composition according to Claim 6, in which Q contains a -C=N(→O)- unit.

11. Rubber composition according to Claim 10, in which Q comprises the unit corresponding to the formula (IV) or (V): 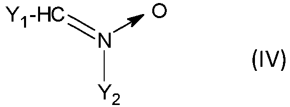 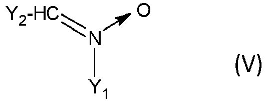 in which:
Y₁ is an aliphatic group, preferably an alkyl group preferably containing from 1 to 12 carbon atoms, or an aromatic group containing from 6 to 20 carbon atoms, preferably an alkylaryl group, more preferably a phenyl or tolyl group,
Y₂, comprising a direct attachment to A, is an aliphatic group, preferably an alkylene group preferably containing from 1 to 12 carbon atoms, or an aromatic group preferably containing from 6 to 20 carbon atoms and comprising, on its benzene nucleus, the direct attachment to A.

12. Rubber composition according to any one of Claims 1 to 11, in which the amount of 1,3-dipolar compound is between 0 and 3 molar equivalents, preferably between 0 and 2 molar equivalents, more preferably between 0 and 1 molar equivalent, more preferably still between 0 and 0.7 molar equivalent, of imidazole ring per 100 moles of monomer units constituting the diene elastomer.

13. Rubber composition according to any one of Claims 1 to 12, in which the diene elastomer is an essentially unsaturated elastomer selected from the group consisting of polybutadienes, polyisoprenes, butadiene copolymers, isoprene copolymers and the mixtures of these elastomers.

14. Rubber composition according to any one of Claims 1 to 13, in which the reinforcing filler comprises an organic filler, preferably a carbon black, or a reinforcing inorganic filler, preferably a silica.

15. Process for preparing a rubber composition based on at least one diene elastomer, a 1,3-dipolar compound, a reinforcing filler and a crosslinking system, which process comprises the following stages:
- adding, during a first "non-productive" stage, to the diene elastomer, the 1,3-dipolar compound, the reinforcing filler and, if appropriate, a coupling agent, by kneading thermomechanically until a maximum temperature of between 130°C and 200°C is reached,
- cooling the combined mixture to a temperature of less than 100°C,
- subsequently incorporating the crosslinking system,
- kneading everything up to a maximum temperature of less than 120°C,
which 1,3-dipolar compound is as defined in Claim 1.

16. Tread which comprises a rubber composition according to any one of Claims 1 to 14.

17. Tyre which comprises a rubber composition according to any one of Claims 1 to 14, preferably in its tread.
